(19) Europäisches Patentamt
European Patent Office
Office européen des brevets

(11) Publication number: **0 428 506 A2**

(12) **EUROPEAN PATENT APPLICATION**

(21) Application number: **91200200.3**

(22) Date of filing: **15.10.86**

(51) Int. Cl.[5]: **C01B 33/34**, B01J 29/28, C07C 5/41, C07C 5/22

This application was filed on 01 - 02 - 1991 as a divisional application to the application mentioned under INID code 60.

(30) Priority: **15.10.85 GB 8525404**

(43) Date of publication of application: **22.05.91 Bulletin 91/21**

(60) Publication number of the earlier application in accordance with Art.76 EPC: **0 219 354**

(84) Designated Contracting States: **AT BE CH DE ES FR GB IT LI LU NL SE**

(71) Applicant: **EXXON CHEMICAL PATENTS INC.**
**200 Park Avenue**
**Florham Park New Jersey 07932(US)**

(72) Inventor: **Verduijn, Johannes Petrus**
**Westersingel 34**
**NL-3202 XL Spijkernisse(NL)**

(74) Representative: **Northover, Robert Frank et al**
**Exxon Chemical Limited Exxon Chemical Technology Centre PO Box 1**
**Abingdon Oxfordshire, OX13 6BB(GB)**

(54) **Zeolite L.**

(57) Zeolite L with a silica/alumina ratio of greater than 6.5, which may be used in catalysis, particularly for aromatisation, is prepared in a synthesis modified by the addition of small amounts of additional metal such as magnesium, calcium, barium, cobalt, zinc, chromium, manganese or nickel.

EP 0 428 506 A2

# IMPROVED ZEOLITE L

This invention relates to a highly crystalline zeolite L, its preparation and use in cataylsis, particularly for aromatization.

Zeolite L has been known for some time as an adsorbant, and in US 3,216,789 is described as an aluminosilicate of the formula:

$0.9 - 1.3\ M_{2/n}O{:}Al_2O_3{:}5.2 - 6.9\ SiO_2{:}yH_2O$

(where M is an exchangeable cation of valence n and y is from 0 to 9) having an x-ray diffraction pattern with the following more significant d(Å) values:

16.1 ± 0.3
7.52 ± 0.04
6.00 ± 0.04
4.57 ± 0.04
4.35 ± 0.04
3.91 ± 0.02
3.47 ± 0.02
3.28 ± 0.02
3.17 ± 0.01
3.07 ± 0.01
2.91 ± 0.01
2.65 ± 0.01
2.46 ± 0.01
2.42 ± 0.01
2.19 ± 0.01

The preparation of zeolite L described in US 3,216,789 comprises crystallizing the zeolite from a reaction mixture comprising mole ratios:

| | |
|---|---|
| $K_2O/(K_2O + Na_2O)$ | 0.33 - 1 |
| $(K_2O + Na_2O)/SiO_2$ | 0.35 - 0.5 |
| $SiO_2/Al_2O_3$ | 10 - 28 |
| $H_2O/(K_2O + Na_2O)$ | 15 - 41 |

The silica to alumina ratio in this reaction mixture is significantly higher than the ratio in the formed zeolite.

British Patent 1,202,511 describes a revised zeolite L preparation using lower proportions of silica in the reaction mixture which comprises mole ratio of reactants as:

| | |
|---|---|
| $K_2O/(K_2O + Na_2O)$ | 0.7 - 1 |
| $(K_2O + Na_2O)/SiO_2$ | 0.23 - 0.35 |
| $SiO_2/Al_2O_3$ | 6.7 - 9.5 |
| $H_2O/(K_2O + Na_2O)$ | 10.5 - 50 |

The ratio $H_2O/(K_2O + Na_2O + SiO_2 + Al_2O_3)$ is preferably not greater than 6 to give a "dry gel".

US 3,867,512 discloses a preparation of zeolite L from a reaction mixture having a molar composition:

| | |
|---|---|
| $K_2O/(K_2O + Na_2O)$ | 0.3 - 1 |
| $(K_2O + Na_2O)/SiO_2$ | 0.3 - 0.6 |
| $SiO_2/Al_2O_3$ | 10 - 40 |
| $H_2O/(K_2O + Na_2O)$ | 15 - 140 |

in which the silica source is a gel having at least 4.5 weight percent water and prepared in a particular manner.

L. Wilkosz in Pr Chem 409 (1974) - Chemical Abstracts, Vol. 90 (1979) 573478 describes the preparation of zeolite L from a synthesis sol prepared by treating a solution containing silica, potassium hydroxide and sodium hydroxide with a second solution containing potassium aluminate, potassium hydroxide and sodium hydroxide and crystallizing for 72 hours at 20 ° C and 122 hours at 100 ° C. The zeolite L product has a $SiO_2$:$Al_2O_3$ ratio of 6.4:1.

G.V. Tsitsishvilli et al in Doklady Akademii NaukSSSR, Vol. 243, No. 2, pp 438-440 (1978) describe the synthesis of zeolite L from alumina-silica gels containing tributylamine. The gels used had the following molar ratios:

| | |
|---|---|
| $SiO_2:Al_2O_3$ | 25 |
| $(K_2O + Na_2O):Al_2O_3$ | 18 |
| $(K_2O + Na_2O):SiO_2$ | 0.72 |
| $H_2O/K_2O + Na_2O$ | 20 |
| $K_2O:Na_2O$ | 0.5 |

Y. Nishiimura in Nippon Kagaku Zasshi 91, 11, 1970, pp 1046-9 describes in general terms zeolite L preparation from a synthesis mixture containing colloidal silica, potassium aluminate and potassium hydroxide having a $SiO_2$:$Al_2O_3$ ratio of 15 - 25, but exemplifies only two synthesis mixtures having the following ratios of components:

$7K_2O:Al_2O_3:20SiO_2:450H_2O$ and

$8K_2O:Al_2O_3:10SiO_2:500H_2O$

Frety et al in C.R. Acad Sc. Paris, t275, Serie c-1215 describes the electron microscopy examination of zeolite L in which particles were said to be observed in the form of slightly deformed cylinders with very variable dimensions.

US 3,298,780 describes a related zeolite known as UJ prepared from an aqueous reactant solution having a composition, expressed as molar ratios of oxides, corresponding to:

$SiO_2/Al_2O_3$ of from 6 to 30

$R_{2/u}O/SiO_2$ of from 0.30 to 0.70, and

$H_2O/R_{2/u}O$ of from 80 to 140

at a temperature between 150 ° F (65.6 ° C) and 325 ° F (162.8 ° C) until the zeolite crystals are formed.

Zeolite UJ is described as having nearly cubic shaped crystals with a crystal size ranging upward from 0.05 micron.

GB 1,393,365 describes a further related zeolite known as AG1 which is prepared by reacting at least one aluminium component, at least one silicon component and at least one alkali metal component, in an aqueous medium, the sole or major silicon component being a water glass having a molar ratio $SiO_2/M_2O$ of 3.5 to 4.0 to give a reaction mixture with oxide molar ratios in one of the following ranges:

| | | |
|---|---|---|
| Range 1 | $SiC_2/Al_2O_3$ | 7 - 14 |
| | $(K_2O + NaO_2)/SiO_2$ | 0.25 - 0.85 |
| | $K_2O/(K_2O + Na_2O)$ | 0.75 - 1 |
| | $H_2O/(K_2O + Na_2C)$ | 25 - 160 |
| Range 2 | $SiC_2/Al_2C_3$ | 14 - 20 |
| | $(K_2O + Na_2O)/SiC_2$ | 0.25 - 0.85 |
| | $K_2C/(K_2O + Na_2O)$ | 0.5 - 1 |
| | $H_2O/(K_2O + Na_2O)$ | 25 - 160 |
| Range 3 | $SiC_2/Al_2C_3$ | 20 - 40 |
| | $(K_2O + Na_2C)/SiO_2$ | 0.25 - 1 |
| | $K_2O/(K_2C + Na_2O)$ | 0.4 - 1 |
| | $H_2O/(K_2O + Na_2O)$ | 25 - 160 |

EP0096479 describes zeolite L crystallites in the form of distinct circular cylinders with a mean diameter of at least 0.5 micron and an aspect ratio of at least 0.5, and their preparation in which an alkaline reaction mixture comprising water, a source of silicon and a source of aluminium with a composition falling within the following molar ratios (expressed as oxides):

| | |
|---|---|
| $M_{2/n}O/SiO_2$ | 0.22 - 0.36 |
| $H_2O/M_2O$ | 25 - 90 |
| $SiO_2/Al_2O_3$ | 6 - 15 |

(wherein M is a cation of valence n, and preferably potassium or a mixture of K + $M^1$ in which $M^1$ is an alkali metal or alkaline earth metal such as sodium, calcium, barium, or rubidium, provided that $K_{2/n}O/M^1{}_2O$ + $K_2O$) is at least 0.7) is heated to a temperature of from at least 75° C and preferably from 100° C to 250° C, more preferably from 120° C to 225° C, to form the desired cylindrical aluminosilicate.

EP0142353 describes a process for the preparation of zeolite L in which an alkaline reaction mixture comprising water, a source of silicon and a source of aluminium with a composition having the following molar ratio (expressed as oxides):

| | |
|---|---|
| $M_{2/n}O/SiO_2$ | 0.22 - 0.30 |
| $H_2O/M_2O$ | 25 - 45 |
| $SiO_2/Al_2O_3$ | 10 - 12 |

(wherein M is as defined hereinbefore) is heated to a temperature of greater than 150° C for a period long enough to form zeolite L.

EP0142354 describes a process for the preparation of zeolite L in which an alkaline reaction mixture comprising water, a source of silicon and a source of aluminium with a composition having the following

molar 5 ratio (expressed in oxides):

| | |
|---|---|
| $M_{2/n}O/SiO_2$ | 0.24 - 0.35 |
| $H_2O/M_2O$ | 35 - 80 |
| $SiO_2/Al_2O_3$ | 5.7 - 7.8 |

(wherein M is as defined hereinbefore) is heated to a temperature of greater than 200 °C to form zeolite L.

Further zeolite L preparations are described in EP0142355, EP0142347, EP0142348 and EP0142349.

It was subsequently found that zeolite L may be used as a catalyst base in aromatization reactions. US4,104,320 discloses dehydrocyclization of aliphatic compounds in the presence of hydrogen using a catalyst comprising zeolite L and a group VIII metal,in which the zeolite L is of the formula:

$M_{9}/n\ (AlO_2)_9(SiO_2)_{27}$

(where M is a cation of valence n) but the silica to alumina ratio may vary from 5 to 7. Zeolite L is described as occuring in the form of cylindrical crystals a few hundred Angstroms in diameter.

East German Patent 88789 discloses dehydrocyclization using a catalyst formed from a zeolite precursor with a silica to alumina ratio of up to 70. Zeollte L is mentioned as a precursor.

European Patent Application Publication 40119 discloses a dehydrocyclization process operating at low pressure

(1 to 7 bars) or low $H_2$/hydrocarbon ratio using a catalyst comprising platinum on a potassium zeolite L. BE 888365 describes dehydrocyclization using a catalyst comprising platinum, rhenium (incorporated in the form of its carbonyl) and sulphur to give an atomic ratio of sulphur to platinum of 0.05 to 0.6 on a zeolitic crystalline aluminosilicate base such as zeolite L. BE792608 discloses the treatment of zeolite L for use as catalyst in isomerization by exchange with ammonium and chromium ions. EP0142351 describes a method for improving the dispersion of noble metal particles on zeolite L to provide an improved reforming catalyst. EP0145289 describes a catalyst comprising zeolite L and highly dispersed particles of a Group VIII metal.

GB2116450 describes a zeolite catalyst comprising a zeolite of the L family, at least one Group VIII metal and an alkaline earth metal selected selected from barium, strontium and calcium. The catalyst is used for reforming, dehydrocyclizing acyclic hydrocarbons, dehydroisomerizing alkylcyclopentanes and dealkylating toluene. Process using such catalyst are described in GB2114150 and GB2142648.

It has now been found that an improved zeolite L, but having a characteristic silica/alumina ratio is particularly valuable for use as a catalyst base in hydrocarbon conversions such as aromatization.

The prior art, and particularly EP 0096479, EP 0142353 and EP 0142354, teaches that in zeolite L synthesis a reduction in alkalinity leads to zeolite W formation, and also to larger crystallites. It has surprisingly now been found that by incorporation of certain additional metals in the synthesis gel zeolite L may be made at low alkalinities and under conditions which normally favour formation of other zeolite species.

The zeolite L of the invention has a relatively high silica/alumina ratio. The cylindrical crystallites described in EP0096479 have silica/alumina ratio of not greater than 6.3, and zeolite L products described therein with higher silica/alumina ratio were in the form of clams or discs (that is, with an aspect ratio of less than 0.5). Thus, the invention comprises zeolite L in the form of cylindrical crystallites having an aspect ratio of at least 0.5 and a silica/alumina ratio of greater than 6.5.

The aspect ratio is the ratio of the axial length of the cylindrical surface 1 to the mean diameter d, and is preferably at least 0.75 and most preferably at least 1.

Preferably the zeolite L comprises cylindrical crystallites wherein at least 80%, more preferably at least 90%, of the basal planes are microscopically flat to within 200 Å, and thus do not exhibit spiral step growths thereon.

The zeolite L of the invention is characterised by its cylindrical morphology. The terms "cylinder" and "cylindrical" are used herein to describe the shape of a cylinder as defined in solid geometry - that is, a solid bounded by a surface generated by a line moving parallel to a fixed line so as to cut a fixed plane curve and by two parallel planes (bases) which cut the surface. The cylinders will generally be circular cylinders, that is, with circular cross-section, but in the context of the invention the cylinders may also exhibit some flattening of the cylindrical surface such that the cross-section has polygonal, and particularly hexagonal character - that is to say, is in the form of a curvilinear hexagon - and the terms "cylinder" and "cylindrical" are used to include such forms.

The zeolite L of the invention displays an x-ray diffraction pattern typical for zeolite L, subject to the

changes in position and intensity of the x-ray lines discussed in EP0096479. Occasionally, additional lines not belonging to the pattern for zeolite L appear in a pattern along with the x-ray lines characteristic of the zeolite. This is an indication that one or more additional crystalline materials are mixed with zeolite L in the sample being tested. It is a preferred feature of the invention that the amount of such additional crystalline materials is minimised in the zeolite material as synthesized. In particular, it is preferred that the synthesis of the zeolite of the invention is conducted so that the amount of zeolite W in the product of the synthesis is minimised. Further, the synthesis of the zeolite of the invention is preferably conducted such that the product of the synthesis is substantially free of any additional crystalline phase giving rise to a line in the x-ray pattern at d (Å) value of 6.28 ± 0.05.

The zeolites of the invention are preferably aluminosilicates and will be described hereinafter in terms of aluminosilicates, though other elemental substitutions are possible, for example aluminium may be substituted by gallium, boron, iron and similar trivalent elements, and silicon may be substituted by elements such as germanium or phosphorus. The aluminosilicates preferably have a composition (expressed in terms of molar ratios of the constituent oxides in anhydrous form) of:

$$(0.9 - 1.3)\ M_{2/n}O:Al_2O_3:xSiO_2$$

wherein M represents one or more cations of valence n, x is from greater than 6.5 to 7.5. The zeolitic materials of the invention have high crystallinity as shown by a well-defined x-ray diffraction pattern (without binder or other diluents present) with sharp peaks.

The exchangeable cation M in general formula I is potassium, but it is possible for a part of M to be replaced by other cations such as alkali metals for example sodium. As described hereinafter, such cations may be introduced during synthesis. The ratio $M_{2/n}O:Al_2O_3$ is preferably from 0.95 to 1.15 and generally above 1.

The aluminosilicate forms of the invention may be hydrated, typically with from 0 to 9 moles of water per mole of $Al_2O_3$. When used as a catalyst base, as described hereinafter, the zeolite of the invention is preferably first calcined to remove water. In normal preparation from aqueous gels a hydrated form is first prepared and this may be dehydrated by heating.

Cylindrical particles have been shown in EP0096479 to have excellent properties of extending catalyst life when used as catalyst bases for aromatization catalysts as compared to the morpologies produced by prior art processes. It is now found that particles of this invention provide a means of extending catalyst life still further and/or giving surprising increases in activity in aromatization and/or in selectivity to aromatic products and/or show increased stability.

It is further surprising feature of the invention that the improved zeolite L with cylindrical morphology may be prepared in the presence of small amounts of additional metals, preferably alkaline earth metals and certain transition metals by controlling the composition of the reaction mixture within certain limits, which in the absence of additional metal do not necessarily result in the desired zeolite L.

The zeolite L of the invention may be prepared by a process for the preparation of zeolite L crystallites in the form of cylinders, in which an alkaline reaction mixture comprising water, a source of an alkali metal, a source of silicon, a source of aluminium and a source of an additional metal $M^{11}$, with a composition falling within the following molar ratios (expressed as oxides):

| | |
|---|---|
| $(M^1_2O + M^{11}_{2/n}O)/SiO_2$ | 0.18 - 0.36 |
| $H_2O/(M^1_2O + M^{11}_{2/n}O)$ | 25 - 90 |
| $SiO_2/Al_2O_3$ | 5 - 15 |
| $M^1_2O/(M^1_2O + M^{11}_{2/n}O)$ | 0.9 - 0.9999 |

(Wherein $M^1$ is an alkali metal, $M^{11}$ is magnesium, calcium, barium, manganese, chromium, cobalt, nickel or zinc cation and n is the valence of $M^{11}$) is heated to a temperature of from at least 75° C and preferably from 100° C to 250° C, more preferably from 120° C to 225° C, to form the zeolite L of the invention.

There are five principal components to the reaction mixture or synthesis gel and thus generally:

aluminium
silicon
alkali metal, preferably potassium

additional metal $M^{11}$
water
and the relative proportions of these components and the chosen reaction conditions are important if the desired zeolite L of the invention is to be obtained.

Zeolite W tends to be formed as a contaminant in zeolite L preparation at some extremes of gel composition. It is advantageous for the zeolite W content of the product to be minimized. The zeolite W content of the product can be monitored by its x-ray diffraction pattern.

A characteristic prominent line 35 in the zeolite W XRD pattern is at 20 = 12.6° (d = 7.09 Å), while a prominent line in the zeolite L XRD pattern is at 28 = 22.7° (d = 3.91 Å). The relative peak intensities of these peaks can be compared to determine the relative proportions of the two zeolite types, since these peaks are not obscured in mixtures of the two zeolites. It is a preferred feature that zeolite of the invention has an XRD pattern in which the peak height ratio (d = 7.09 Å)/ (d = 3.91Å) is not greater than 0.2. Very preferably the product is substantially free of zeolite W as evidenced by an absence of the XRD pattern of a line at a d spacing of 7.09 Å. As described in EP0096479, it was previously thought necessary to avoid contamination for the reaction mixture to comprise the reactants in the following molar ratios:

$$M_{2/n}O/SiO_2 = > 0.25$$
$$H_2O/M_{2/n}O = < 65$$
$$SiO_2/Al_2O_3 = 7.5 - 10.5$$

It is a surprising feature of the present invention that a highly crystalline product of the invention, substantially free of zeolite W may be obtained from a reaction mixture outside the ranges taught by the prior art, and specifically in which:
$M_{2/n}O/SiO_2 < 0.25$ and/or
$H_2O/M_{2/n}o > 65$ and/or

$SiO_2/Al_2O_3 = 5 - 7.5$

by selecting a reaction mixture such that M is a mixture of $M^1 + M^{11}$ where:

$M^1{}_2O/(M^1{}_2O + M^{11}{}_{2/n}O = 0.900 - 0.9999$

The alkali metal $M^1$ is very preferably potassium (K), but may be a mixture of potassium with other alkali metals, for example sodium. It is a further surprising feature of the invention that a greater degree of replacement of potassium by other alkali metals in possible in the presence of the additional metal without significant amounts of zeolite W being formed in the Zeolite L product. EP0096479 indicates that the preferred maximum amount of alkali metal other than potassium is 30 mole % of the total alkali metal content. We have found that at this level of other alkali metal and even at greater levels the tendency to form zeolite W may be substantially completely surpressed by the presence of the additional metal $M^{11}$

Thus, the preferred zeolites of the invention may be obtained within the following preferred ranges:

| | |
|---|---|
| $(M^1{}_2O + M^{11}{}_{2/n}O)/SiO_2$ | 0.18 - 0.26 |
| $H_2O/(M^1{}_2O + M^{11}{}_{2/n}O)$ | 50 - 90 |
| $SiO_2/Al_2O_3$ | 6 - 12 |
| $M^1{}_2O/(M^1{}_2O + M^{11}{}_{2/n}O)$ | 0.959 - 0.9999 |

where $M^1$ is potassium or a mixture of potassium and a second alkali metal $M^2$ and $K_2O/K_2O + M_2O$ = 0.5-1.

The amount of the additional metal $M^{11}$may be very low, only a few ppm of the reaction mixture, and yet still have an effect in promoting zeolite L formation, forming smaller and/or more cylindrical zeolite L particles and/or promoting the properties of the zeolite L product.

In addition to varying the proportions of the reactants in the reaction mixture, it is possible to vary the reaction conditions and in particular the crystallisation temperature. By using different temperatures, it may be possible to deviate further from the preferred composition defined above and yet still obtain the desired product. In general, within the broad reactant ratios defined for the process of the invention, a higher crystallisation temperature enables the silicon content to be lowered and/or the water content to be lowered and/or the potassium content (and thus the alkalinity) to be raised. By contrast, operating at lower temperatures tends to decrease the nucleation rate which can be countered by lowering the alkalinity and/or by increasing the water content and/or by introducing seeds of preformed zeolite L. Increasing the alumina content enables higher yields of zeolite L to be obtained, whereas the prior art teaches that this leads to zeolite W formation.

The additional metal $M^{11}$ may be introduced as any convenient compound such as an oxide, carbonate, silicate hydroxide or sulphate. Barium sulphate has been found an effective source of barium even though not soluble in the reaction medium. The additional metal $M^{11}$ is preferably magnesium, calcium, barium, zinc or cobalt though chromium manganese or nickel may also be employed.

The function of the additional metal is not wholly understood but at least some of the metals are believed to form highly insoluble silicates which may form very fine suspensions of silicate particles which will function as nuclei or seeds for zeolite L formation. Thus additional metals forming highly insoluble silicates are preferred.

In the synthesis of all zeolitic materials of the invention, the source of silicon for the reaction mixture is generally silica, and this is usually most conveniently in the form of a colloidal suspension of silica such as Ludox BS 40 available from E.I. Dupont de Nemours and Co. Colloidal silica sols are preferred since they result in less contaminating phases. However, other forms such as silicates may be used.

The source of aluminium may be an alumina introduced into the reaction medium as, for example, $Al_2O_3.3H_2O$, previously dissolved in alkali. However, it is also possible to introduce aluminium in the form of the metal, which is dissolved in alkali.

The aluminosilicates of the invention are preferably obtained from reaction mixtures containing potassium. This potassium is preferably introduced as potassium hydroxide.

The product of the processes described above is a mixed cation form of the zeolite containing alkali metal, preferably potassium, and metal $M^{11}$. The molar ratio of $K_2O/(K_2O + M^{11}{}_{2/n}O)$ in the product of the invention is preferably greater than 0.95, more preferably greater than 0.98. The amount of the cation $M^{11}$ in the zeolite is preferably less than 0.1 wt% of the zeolite L, and may be below 0.05 wt% of the zeolite L. By ion exchange of the product in the manner conventional to zeolite chemistry, other cations can be introduced. However it is a surprising feature of the invention that the cation $M^{11}$ cannot be completely replaced by ion exchange indicating that some of them at least of the $M^{11}$ cations are in non-exchangeable sites in the zeolite L structure.

Within the ranges specified hereinbefore for the composition of the reaction mixture, it is possible to choose ratios of oxides and alkalinity to given particular forms of the aluminosilicate product. The $SiO_2/Al_2O_3$ ratio in the reaction mixture may vary over a wide range but the $SiO_2/Al_2O_3$ ratio in the product preferably lies in a relatively narrow range of greater than 6.5 to 7.4. The higher the $SiO_2/Al_2O_3$ ratio in the reaction mixture, the higher the ratio in the product. Also, decreasing alkalinity ($OH^-/SiO_2$) tends to increase the $SiO_2/Al_2O_3$ ratio in the formed product. Dilution of the reaction mixture with water and thus increasing the $H_2O/K_2O$ ratio also tends to increase the $SiO_2/Al_2O_3$ ratio in the product.

Particle size is also affected by the composition of the reaction mixture and the nature of the raw materials used. Generally, the particles formed are in the range of from 0.05 to 4.0 $\mu$, but the use of the metal $M^{11}$ tends to favour small particles, despite the low alkalinity of the synthesis gel. However, even in the presence of $M^{11}$ larger, but still small, particle sizes are favoured by lower alkalinity. Higher dilution and high temperatures tend to favour formation of particles with an increased l/d ratio.

Crystallisation time is related to the crystallisation temperature. The cystallisation is preferably carried out in the region of 150°C and at this temperature the crystallisation time may be from 24 to 96 hours, typically from 48 to 72 hours. Lower temperatures may require much longer times to achieve good yield of the desired product, whereas times of less than 24 hours are possible when higher temperatures are used. A time of 8 to 15 hours is typical for a temperature of 200°C or greater.

The crystallisation is generally carried out in a sealed autoclave and thus at autogenous pressure. It is generally inconvenient, although possible, to employ higher pressures. Lower pressure will require longer crystallisation times.

Following the preparation as described above the zeolite L may be separated, washed and dried in the normal manner.

The products of the processes of the invention described hereinbefore are preferably substantially free

from contaminant crystalline and amorphous materials. However, in employing these products in catalytic applications it may be desired to combine them with additional crystalline or amorphous materials and this invention extends to such combinations.

We have found that the zeolite L of the invention is an excellent catalyst base and may be used in a wide variety of catalytic reactions. The particular morphology of the crystals appears to result in a particular stable base for catalytically active metals with a surprising resistance to metal catalyst deactivation. In addition, the zeolite L of the invention has displayed low acidity which makes it especially suited to catalytic applications where a low acid site strength is advantageous such as aromatisation.

The catalytically-active metal(s) may be, for example, a Group VIII metal such as platinum, tin, or germanium as described in US 4,104,320, or a combination of platinum and rhenium as described in GB 2,004,764 or BE 888365. In the latter case, the catalyst may for appropriate circumstances also incorporate halogen as described in US 4,165,276, silver as described in US 4,295,959 and US 4,206,040, cadmium as described in US 4,295,960 and US 4,231,897 or sulphur as described in GB 1,600,927.

We have found a particularly advantageous catalyst composition to incorporate from 0.1 to 6.0 weight%, preferably from 0.1 to 1.5 weight% platinum or palladium, since this gives excellent results in aromatisation. From 0.4 to 1.2 weight% platinum is particularly preferred, especially in conjunction with the potassium form of the aluminosilicate. The invention extends to catalysts comprising the zeolitic material and a catalytically-active metal.

It may also be useful to incorporate into the catalyst of the invention one or more materials substantially inert under the conditions in which the catalyst is to be employed to act as a binder. Such binders may also act to improve the resistance of the catalyst to temperature, pressure and attrition.

The zeolite L of the invention may be used in a process for the conversion of a hydrocarbon feed in which the feed is contacted with a catalyst as described above under appropriate conditions to bring about the desired conversion. They may, for example, be useful in reactions involving aromatisation and/or dehydrocyclisation and/or isomerisation and/or dehydrogenation reaction. They are particularly useful in a process for the dehydrocyclisation and/or isomerisation of aliphatic hydrocarbons in which the hydrocarbons are contacted at a temperature of from 370 to 600 °C, preferably 430 to 550 °C, with a catalyst comprising zeolite L of the invention, preferably having at least 90% of the exchangeable cations M as alkali metal ions, and incorporating at least one Group VIII metal having dehydrogenating activity, so as to convert at least part of the aliphatic hydrocarbons into aromatic hydrocarbons.

The aliphatic hydrocarbons may be straight or branched chain acyclic hydrocarbons, and particularly paraffins such as hexane, although mixtures of hydrocarbons may also be used such as paraffin fractions containing a range of alkanes possibly with minor amounts of other hydrocarbons. Cycloaliphatic hydrocarbon such as methylcyclopentane may also be used. In a preferred aspect the feed to a process for preparing aromatic hydrocarbons and particularly benzene comprises hexanes. The temperature of the catalytic reaction may be from 370 to 600 °C, preferably 430 to 550 °C and preferably pressures in excess of atmospheric are used, for example up to 2000 KPa, more preferably 500 to 1000 KPa. Hydrogen is employed in the formation of aromatic hydrocarbons preferably with a hydrogen to feed ratio of less than 10.

The process is preferably otherwise carried out in the manner described in US 4,104,320, BE 888365, EP 40119, EP 0142351, EP 0145289 or EP0142352.

It has been found that use of the zeolite L of the invention in this way enables greatly improved catalyst lifetimes to be achieved as compared to the lifetime obtained with a conventionally prepared zeolite.

The invention will now be described in more detail, though only by way of illustration, in the following examples and evaluations.

Comparative Example 1: Preparation of Zeolite L

Zeolite L was prepared according to the procedure of EP 0096479. A synthesis gel was prepared having the following composition expressed in moles of pure oxide:

$2.60K_2O:Al_2O_3: 10SiO_2:160H_2O$

This gel was prepared as follows:
The aluminium hydroxide was dissolved by boiling in an aqueous solution of potassium hydroxide pellets (86% pure KOH) to form Solution A. After dissolution any water loss was corrected. A separate solution,

Solution B, was prepared by diluting colloidal silica (Ludox HS 40) with water.

Solutions A and B were mixed for two minutes to form a gel, and just before the gel became fully stiff, it was transferred to a Teflon-lined autoclave, preheated to 150 °C and held at that temperature for 72 hours to bring about crystallisation.

The formed zeolite L was highly crystalline with a typical zeolite L x-ray diffraction (XRD) pattern. Scanning electron micrographs (SEM) show the product to be formed solely of cylindrical crystals having a mean diameter of 1 to 2 microns, an aspect ratio (l/d) of

0.5 - 1 and an l/h ratio of 0.65 - 0.95. The $SiO_2$:$Al_2O_3$ ratio in the product was 6.3.

Comparative Example 2-6:

Variation of potassium content

As shown in Table 1, the variation of the amount of potassium in the case where M=K was also investigated. Variation of potassium content from 2.41 moles $K_2O$ (Example 3) to 2.75 moles $K_2O$ (Example 2) gave zeolite L with a cylindrical form, but with l/h < 1. Example 4 gave zeolite L with a morphology intermediate a clam shape and the cylinder shape - that is, l/h was very much less than 1.

A low potassium content of 2.15 moles $K_2O$ (Example 5) gave a product with low crystallinity. A high potassium content of 3.4 moles $K_2O$ (Example 6) gave a clam-shaped product.

Example 7: Preparation of

zeolite L of the invention

The procedure of Example 1 was modified by the addition of certain metal cations to the synthesis gel.

Solution A was prepared from:

| | |
|---|---|
| KOH (87.5% KOH) pellets | 30.81 g |
| Aluminium hydroxide | 15.61 g |
| Water | 49.82 g |
| Additional water | 25.39 g |

The aluminium hydroxide powder was dissolved in the potassium hydroxide solution by boiling. After cooling to ambient temperature, the water loss was corrected.

Solution B was prepared from:

| | |
|---|---|
| Barium hydroxide $Ba(OH)_2.8H_2O$ | 0.17 g |
| Silica (Ludox HS 40) | 150.16 g |
| Water | 120.32 g |
| Seeds of zeolite L from Example 1 | 0.75 g |

Barium hydroxide powder was added to the colloidal silica solution, which gelled on mixing. The synthesis gel was seeded with pre-formed zeolite L crystallites to enhance the crystallization process, but

such seeding is not essential.

Solution A was added to Solution B and mixed for 5 minutes to form the synthesis gel. This had the composition (in moles of oxides):

$2.4K_2O:0.005BaO:Al_2O_3:10SiO_2:164H_2O$

Crystallisation was conducted at 175 °C for 73 hours. The product was washed 4 times with 500 $cm^3$ water. The pH of the water from the last washing was 10.6. The product was dried at 125 °C and the results are given in Table 3. The barium content of the product was measured to be about 300 ppm by atomic adsorption spectroscopy, and about 80 ppm by ion plasma emmission spectroscopy.

Examples 8 - 11: Preparation of

zeolite L of the invention

The procedure of Example 7 was repeated with different amounts of barium present and using magnesium and calcium in place of barium, with and without seeding. The synthesis conditions are set out in Table 2 and the details of the products are given in Table 3.

**TABLE 1**

| Comparative Example | Gel Composition (moles) $K_2O$ | $Al_2O_3$ | $SiO_2$ | $H_2O$ | Product (wt%) $K_2O$ | $Al_2O_3$ | $SiO_2$ | $H_2O$ | |
|---|---|---|---|---|---|---|---|---|---|
| 2 | 2.75 | 1 | 10 | 160 | 6.7 | 2.7 | 15.6 | 75.0 | cylindrical zeolite L (l/h < 1) |
| 3 | 2.41 | 1 | 10 | 160 | 5.9 | 2.7 | 15.8 | 75.6 | cylindrical zeolite L (l/h < 1) |
| 4 | 3.01 | 1 | 10 | 160 | 7.3 | 2.6 | 15.5 | 74.5 | clam/cylinder zeolite L (l/h << 1) |
| 5 | 2.15 | 1 | 10 | 160 | 5.3 | 2.7 | 15.9 | 76.1 | low crystallinity product |
| 6 | 3.4 | 1 | 10 | 160 | 8.2 | 2.6 | 15.4 | 73.8 | clam-shaped zeolite L |

TABLE 2

| Example | Synthesis Gel (moles) | | | | | | Crystallisation Conditions | | |
|---|---|---|---|---|---|---|---|---|---|
| | $M^{11}$ | $K_{2/n}O$ | $M^{11}{}_2O$ | $Al_2O_3$ | $SiO_2$ | $H_2O$ | Temp(°C) | Time (hrs) | Gel Seeded |
| 7 | Ba | 2.40 | 0.005 | | | 164 | 175 | 73 | Yes |
| 8 | Mg | 2.29 | 0.1 | 1 | 10 | 161 | 150 | 68 | yes |
| 9 | Mg | 2.59 | 0.1 | 1 | 10 | 163 | 150 | 65 | yes |
| 10 | Ca | 2.58 | 0.1 | 1 | 10 | 162 | 150 | 65 | yes |
| 11 | Ba | 2.10 | 0.005 | 1 | 10 | 161 | 175 | 77 | no |

TABLE 3

| Example | ...duct ...les) ...) | $M^{11}_{2/n}O$ | $Al_2O_3$ | $SiO_2$ | Crystallinity (relative to Ex. 1) % | W/L a) ratio | Zeolite Type | Morphology | Mean Dia( μ) | Aspect Ratio L/d | L/h |
|---|---|---|---|---|---|---|---|---|---|---|---|
| | 04 | 0.0001 | 1 | 6.7 | 99 | <0.01 | L | cylinder | 0.3-0.5 | 1-3 | 1 |
| | 07 | 0.04 | 1 | 6.9 | 92 | <0.01 | L | cylinder | 0.3-0.5 | 1-1.5 | 0.85-1 |
| | .11 | 0.04 | 1 | 6.8 | 84 | <0.01 | L | cylinder | 0.2-0.3 | 0.8-1 | ? |
| | .08 | 0.05 | 1 | 7.0 | 62 | <0.01 | L | cylinder | 0.2-0.4 | | |
| | .98 | 0.003 | 1 | 6.7 | 76 | 0.45 | L | cylinder | 0.3-0.5 | 1-4 | 1 |

* indicates that the parameter could not be determined at the resolution of the microscopic analysis used.

...atio XRD peak intensity of zeolite W at d = 7.09Å/ zeolite L at d = 22.7 Å

The products obtained are in Examples 8 to 11 - small cylindrical particles of zeolite L.

Example 12: Unseeded synthesis The procedure of Example 7 was repeated but without the addition of seeds. A substantially similar zeolite L product was obtained with the characteristics and properties set out in Example 7.

Evaluation: Aromatisation

The performance of certain of the zeolite L samples of the examples as a catalyst base in aromatisation was compared to products of EP0096479. In each case, a catalyst was prepared by impregnatina the base with 0.64 weight% platinum by an incipient wetness technique in which the base was dried at 140 °C overnight then added to a 1.68 wt% solution of platinum tetraamine dichloride monohydrate, stirred and aged at room temperature for 30 minutes, then dried in a vacuum oven at 1200 C for 4 hours. The impregnated sample was dried at 110 °C for 4 hours, and then pelletised, crushed to 16-45 mesh (US seive size) and loaded into a vertical tubular reactor, calcined in air (substantially water-free) at 480 °C, and then reduced in hydrogen at 510 °C.

An aromatisation screening test was carried out at a temperature of 510 °C and 738 KPa (107 psig) pressure with a $C_6$ mixed feed comprising:

| Component | Weight % |
|---|---|
| iso-$C_6$ (3-methyl-pentane) | 60 |
| n - $C_6$ | 40 |

at a weight hourly space velocity of 8.0 w/w $hr^{-1}$ and in the presence of hydrogen, the $H_2$:hydrocarbon ratio being 4. This is an accelerated test which provides a good correlation with catalyst performance in commercial aromatisation. The results are given in Table 4.

A catalyst using the zeolite of Example 7 as its catalyst base is compared with a catalyst using a zeolite L prepared according to EP0096479 with cylindrical particles (d = 0.7-1.4 $\mu$, l/d = 0.5-1, l/h = 0.67), an $SiO_2/Al_2O_3$ ratio of 5.8 and containing no additional metal $M^{11}$, hereinafter referred to as "Comparison"

**TABLE 4**

| | Example 7 | | Comparison | |
|---|---|---|---|---|
| Time On Stream (hr) | 22 | 50 | 22 | 50 |
| $C_6$ Conversion (%) | 81.6 | 77.2 | 74.9 | 68.4 |
| Benzene Yield (%) | 60.2 | 57.7 | 51.5 | 46.6 |
| Benzene Selectivity (%) | 73.8 | 74.8 | 68.7 | 68.1 |

A 16% (relative) higher benzene yield was observed with the invention whereas no changes in $C_4$ - cracking pattern were observed. The higher aromatics yield is to improved activity (conversion) and to improved selectivity.

Further aromatisation screening tests were carried out on catalysts prepared using the following materials as substrate.

(a) zeolite L of Example 12;

(b) zeolite L of Example 12, exchanged with an aqueous IN potassium nitrate solution for 24 hours at ambient temperature and then dried at 120°C for four hours prior to impregnation with platinum as described above - the Ba content is reduced to 50 ppm (as measured by ion plasma emmission spectroscopy);

(c) Comparison, as defined above;

(d) Comparison, exchanged with barium as described in Example 1 of GB 2116450 to give a Ba content of 100 ppm as measured by ion plasma emmission spectroscopy, prior to impregnation with platinum as described above.

Table 5

| | Example 12, (Example 7 Scale-up) | Example 12 -K exchanged | Comparison | Comparison Ba exchanged |
|---|---|---|---|---|
| Time on stream (hr) | 23 | 23 | 23 | 23 |
| $C_6$ Conversion (%) | 78.7 | 76.3 | 74.9 | 70.1 |
| Benzene yield (%) | 58.2 | 56.9 | 51.2 | 48.8 |
| Benzene selectivity (%) | 74.0 | 74.6 | 68.4 | 69.6 |

These results show that the zeolite L of the invention is better in terms of conversion, yield and selectivity than the zeolite L described in EP 0096749, in this catalytic application. Exchanging the zeolite L of the invention did not remove all barium from the sample, showing that the barium is not all in an exchangeable form, but by removing the exchangeable barium the advantage over the zeolite L of EP 0096479 is not lost. Furthermore, carrying out the barium exchange procedure of GB 2116450 on the zeolite L of EP 0096479 did not improve the performance of the zeolite L in the terms of conversion or benzene yield, but rather made it worse, and the change in benzene selectivity is relatively small. This comparison shows that the advantage of the zeolite L of the invention does not lie in barium exchange as described in GB 2116450.

## Claims

1. Zeolite L in the form of cylindrical crystallites having an aspect ratio of at least 0.5 characterised in that the crystallites have a silica/alumina ratio of greater than 6.5.

2. Zeolite L as claimed in claim 1, which contains a cation $M^{11}$ (wherein $M^{11}$ is a cation of magnesium, calcium, barium, manganese, chromium, cobalt, nickel or zinc) in an amount of less than 0.1 wt% of the zeolite L.

3. Zeolite L as claimed in claim 2, in which at least part of the cation $M^{11}$ is at non-exchangeable sites within the structure.

4. A catalyst comprising zeolite L and a catalytically active metal characterised in that the zeolite L is as claimed in any of claims 1 to 3.

**5.** A catalyst as claimed in claim 4, which comprises from 0.1 to 6.0 weight % platinum.

**6.** A process for the dehydrocyclisation and/or isomerisation of aliphatic hydrocarbons in which the hydrocarbons are contacted at a temperature of from 370 °C to 600 °C with a catalyst so as to convert at least part of the aliphatic hydrocarbons into aromatic hydrocarbons, characterised in that the catalyst is as claimed in claim 4 or claim 5.

**7.** A process as claimed in claim 6, in which a feed comprising hexanes is contacted with the catalyst at a temperature of from 430 °C to 550 °C and a pressure of 500 to 1000kPa, together with hydrogen such that the hydrogen to feed weight ratio is less than 10, so as to convert the hexanes at least in part to benzene.